# EUROPÄISCHE PATENTSCHRIFT

(11) **EP 2 445 391 B1**
(45) Veröffentlichungstag und Bekanntmachung des Hinweises auf die Patenterteilung: **01.05.2013**
(21) Anmeldenummer: 10723543.4
(22) Anmeldetag: 09.06.2010
(51) Int. Cl.: A61B 5/00

(54) **SENSOR FÜR IN-VIVO MESSUNGEN**
SENSOR FOR IN-VIVO MEASUREMENTS
CAPTEUR POUR MESURES IN-VIVO

(30) Priorität: 23.06.2009 EP 09008183
(43) Veröffentlichungstag der Anmeldung: 02.05.2012
(73) Patentinhaber: Roche Diagnostics GmbH, 68305 Mannheim (DE); F.Hoffmann-La Roche AG, 4070 Basel (CH)
(72) Erfinder: KUBE, Oliver, 67549 Worms (DE); RITTINGHAUS, Andrea, 69239 Neckarsteinbach (DE)
(74) Vertreter: Twelmeier Mommer & Partner
(86) Internationale Anmeldenummer: PCT/EP2010/003451
(87) Internationale Veröffentlichungsnummer: WO 2010/149269

(56) Entgegenhaltungen:
- EP-A2- 0 876 823
- US-A- 5 390 671
- US-B2- 7 381 184

## Beschreibung

Die Erfindung geht aus von einem Sensor für in-vivo Messungen mit den im Oberbegriff des Anspruchs 1 angegebenen Merkmalen. Ein derartiger Sensor ist aus der US 5,390,671 bekannt.

Derartige Sensoren ermöglichen eine elektrochemische Messung von Analyten in einem menschlichen oder tierischen Körper, beispielsweise von Glucose oder Lactat. Dazu wird ein auf einem als Sensorschaft ausgebildeten distalen Bereich eines Substrats angeordnetes Elektrodensystem in den Körper eines Patienten eingebracht, so dass elektrochemisch eine transkutane Messung durchgeführt werden kann. Dieser Vorgang wird häufig als Insertion des Sensors bezeichnet. In der Regel wird der Sensorschaft mit einer geschlitzten Kanüle in den Körper eines Patienten eingestochen, die nach der Insertion aus dem Körper des Patienten herausgezogen werden kann, so dass der Sensorschaft mit den Elektroden im Körper des Patienten stecken bleibt. Ein proximaler Bereich des Substrats ragt nach der insertion aus dem Körper heraus und bildet einen Sensorkopf zum Anschließen des Sensors an ein Messgerät. Der Sensorkopf trägt metallisierte Flächen mit Kontaktfeldern und ist meist als ein Steckteil ausgebildet, so dass zum Anschließen des Sensors an ein Mess- oder Auswertegerät eine Kupplung oder Buchse mit einem passenden Schlitz auf das Steckteil aufgesteckt werden kann. Die Anordnung der Kontaktfelder gibt dabei die Steckrichtung vor, in der ein Steckverbinder zum Anschließen des Sensors auf den Sensorkopf aufgesteckt wird.

Wie alle medizinischen Geräte müssen Sensoren zur in-vivo Messung höchsten Anforderungen an die Zuverlässigkeit genügen. Darüber hinaus sollen die Sensoren möglichst leicht handhabbar sein, so dass sie idealerweise auch von medizinischen Laien verwendet werden können. Ferner soll der Gebrauch eines Sensors für Patienten mit möglichst geringen Schmerzen verbunden sein.

Aufgabe der Erfindung ist es, einen Weg aufzuzeigen, wie diese Anforderungen besser erfüllt werden können.

Diese Aufgabe wird durch einen Sensor mit den im Anspruch 1 angegebenen Merkmalen sowie durch ein System gemäß Anspruch 15 gelöst. Vorteilhafte Weiterbildungen der Erfindungen sind Gegenstand von Unteransprüchen.

Bei einem erfindungsgemäßen Sensor steht der Sensorkopf seitlich von dem Sensorschaft ab, so dass die Kontaktfelder seitlich von dem Sensorschaft angeordnet sind. Die durch die Anordnung der Kontaktfelder vorgegebene Steckrichtung ist vorteilhaft quer zur Längsrichtung des Sensorschafts.

Zum Anschließen des Sensors an ein Mess- oder Auswertegerät kann deshalb eine Steckverbindung durch eine quer zu einem von der Kanüle erzeugten Einstichkanal und entlang der Hautoberfläche eines Patienten verlaufende Steckbewegung geschlossen werden. Beispielsweise kann ein Kupplungsteil, das einen zu dem Sensorkopf passenden Schlitz hat, auf den Sensorkopf aufgesteckt werden. Möglich ist es aber auch, dass der Sensorkopf in einem Stützteil angeordnet ist, das zusammen mit dem Sensorkopf einen elektrischen Steckverbinder ausbildet.

Bei einem erfindungsgemäßen Sensor ist deshalb keine Kraft in Längsrichtung des Einstichkanals erforderlich, um diesen an ein Mess- oder Auswertegerät anzuschließen. Demzufolge kann die Gefahr vermieden werden, dass die Kanüle oder der Sensor durch eine zum Schließen einer Steckverbindung erforderliche Kraft unbeabsichtigt noch tiefer in den Körper eines Patienten gedrückt und somit unnötige Schmerzen verursacht werden. Vorteilhaft ist deshalb der Gebrauch eines erfindungsgemäßen Sensors für einen Patienten mit weniger Schmerzen verbunden.

Zudem wird das Anschließen eines erfindungsgemäßen Sensors an ein Messgerät von Patienten auch aus psychologischen Gründen als angenehmer empfunden. Das Ausüben einer Kraft in Stichrichtung eines im eigenen Körper steckenden Sensors wird instinktiv als bedrohlich und deshalb unangenehm empfunden. Mit einem erfindungsgemäßen Sensor kann dieses Problem vermieden werden. Dies führt dazu, dass Patienten, die den Sensor selbstständig an ein Messgerät anschließen, dabei psychologisch nicht gehemmt sind und deshalb eine Steckverbindung leichter fehlerfrei vornehmen können. Ein erfindungsgemäßer Sensor hat deshalb auch den Vorteil, Messergebnisse mit einer erhöhten Zuverlässigkeit zu liefern.

Ein wichtiger Vorteil eines erfindungsgemäßen Sensors ist insbesondere auch, dass man das Substrat zum Anschließen des Sensors an ein Messgerät nicht zu biegen braucht. Indem die Kontaktierung des Sensors von Biegekräften entlastet wird, kann auch Zuverlässigkeit der Steckverbindung erhöht und der Aufbau des das Steckteil aufnehmenden Kupplungsteils vereinfacht werden.

Das Substrat eines erfindungsgemäßen Sensors kann biegsam ausgebildet sein, beispielsweise indem es durch Ausschneiden aus einer Kunststofffolie hergestellt wird. Ein biegsames Substrat hat den Vorteil, dass sich der Sensor im Körper eines Patienten an dessen Bewegungen anpassen kann. Die Angabe, dass das Substrat eines erfindungsgemäßen Sensors flach ist, bezieht sich deshalb auf einen neuen, ungebrauchten Sensor, auf dessen Substrat keine Biegekräfte einwirken. In entsprechende Weise bezieht sich auch die Angabe, dass das Steckteil eine Steckrichtung vorgibt, die mit der Längsrichtung der Kanüle einen spitzen Winkel einschließt, auf einen neuen, ungebrauchten Sensor im kräftefreien Zustand.

Der Sensorkopf eines erfindungsgemäßen Sensors kann als ein Steckteil ausgebildet sein. Zum Anschließen des Sensors an ein Messgerät kann der Sensorkopf dann von einem Benutzer in einen Schlitz einer Buchse oder eines Kupplers gesteckt und auf diese Weise eine Steckverbindung geschlossen werden. Der Sensorkopf kann aber auch von einem Hersteller mit einem Steckteil verbunden werden, das von einem Benutzer zum Anschließen des Sensors mit einem dazu passenden Steckverbindungsteil zusammengesteckt wird.

Bei einem ungebrauchten Sensor ist das Substrat bevorzugt hochkant in dem Schlitz einer Kanüle angeordnet. Mit der Kanüle wird im Körper eines Patienten ein Einstichkanal erzeugt. Die die Kanüle kann nach einem Einstich entfernt werden, wobei das Substrat in dem Einstichkanal stecken bleibt. Nach der Insertion ist die von dem bevorzugt als Steckteil ausgebildeten Sensorkopf vorgegebene Steckrichtung deshalb im kräftefreien Zustand quer zu dem Einstichkanal.

Bei einem erfindungsgemäßen Sensor wird vorteilhaft ein flaches Substrat verwendet, das mit geringem Aufwand beispielsweise dadurch hergestellt werden, dass es aus einem Kunststoffblatt ausgeschnitten wird. In einer Seitenansicht, also mit Blickrichtung senkrecht auf die Schmalseiten des Substrats, hat das Substrat dann die Form eines geradlinigen Streifens. Ein von dem Substrat als Sensorkopf gebildetes Steckteil kann kostengünstig nach dem Prinzip einer Steckkarte ausgebildet werden. Dazu können auf dem Steckteil bzw. dem das Steckteil bildenden Abschnitt des Substrates zur Kontaktgabe metallisierte Flächen als Kontaktfelder angeordnet werden, die über Leiterbahnen jeweils mit einer der Elektroden verbunden sind.

Die Elektroden und Kontaktfelder können ähnlich wie bei einer Leiterplatte auf einer Oberseite oder Unterseite des Substrats angeordnet sein. Dabei können sich alle Elektroden und Kontaktfelder auf einer einzigen Seite des Substrats befinden, die dann üblicherweise als Oberseite bezeichnet wird. Möglich ist es aber auch, dass sowohl auf der Oberseite als auf der Unterseite Elektroden und/oder Kontaktfelder angeordnet sind. Bevorzugt ist dabei ein Kontaktfeld auf der Oberseite elektrisch leitend mit einem Kontaktfeld auf der Unterseite verbunden, um die Kontaktierung zu verbessern. Möglich ist es aber auch, dass die Kontaktfelder auf der Oberseite und auf der Unterseite jeweils mit unterschiedlichen Elektroden verbunden sind, so dass sich durch Ausnutzung von Ober- und Unterseite ein besonders kompaktes Design verwirklichen lässt.

Der Sensorkopf eines erfindungsgemäßen Sensors ist bevorzugt länglich ausgebildet und die Längsrichtung des Sensorkopfs quer zur Längsrichtung des Sensorschafts. Bevorzugt ist ferner, dass der Sensorkopf breiter als der Sensorschaft ist.

Der Sensorkopf kann sich senkrecht zur Längsrichtung des Sensorschafts erstrecken, so dass die Leiterbahnen auf dem Sensorkopf senkrecht zur Längsrichtung des Sensorschafts verlaufen. Auf diese Weise lässt sich ein Steckteil bilden, das eine senkrecht zur Längsrichtung des Sensorschafts verlaufende Steckrichtung vorgibt. Allerdings werden Sensoren für in vivo Messungen in der Regel nicht senkrecht zur Hautoberfläche in den Körper eines Patienten, sondern schräg dazu eingestochen. Bevorzugt erstreckt sich der Sensorkopf deshalb schräg zur Längsrichtung des Sensorschafts und gibt eine Steckrichtung vor, die schräg zur Längsrichtung des Sensorschafts verläuft. Hierfür ist es vorteilhaft, wenn die Leiterbahnen auf dem Sensorkopf schräg zur Längsrichtung des Sensorschafts verlaufen. Wenn der Sensor schräg zur Hautoberfläche in den Körper eines Patienten eingestochen wird, kann die Steckverbindung dann nämlich ergonomisch vorteilhaft geschlossen werden, indem ein Steckverbinder zum Kontaktieren des Sensorkopfes näherungsweise parallel zur Hautoberfläche bewegt wird.

Weitere Einzelheiten und Vorteile der Erfindung werden an einem Ausführungsbeispiel unter Bezugnahme auf die beigefügten Zeichnungen erläutert. Gleiche und einander entsprechende Komponenten sind dabei mit übereinstimmenden Bezugszahlen bezeichnet. Es zeigen:
- Figur 1: einen erfindungsgemäßen Sensor;
- Figur 2: den in Figur 1 dargestellten Sensor ohne Kanüle;
- Figur 3: eine Seitenansicht zu Figur 2;
- Figur 4: eine Querschnittsansicht zu Figur 1;
- Figur 5: eine Ansicht der Kanülenspitze;
- Figur 6: ein weiteres Ausführungsbeispiel eines erfindungsgemäßen Sensors;
- Figur 7: das in Figur 2 gezeigte Ausführungsbeispiel mit einem Stützteil;
- Figur 8: eine Schnittansicht zu Figur 7;
- Figur 9: einen Steckverbinder zu dem in Figur 7 gezeigten Ausführungsbeispiel;
- Figur 10: das in Figur 7 gezeigte Ausführungsbeispiel zusammen mit einer Trägereinheit.

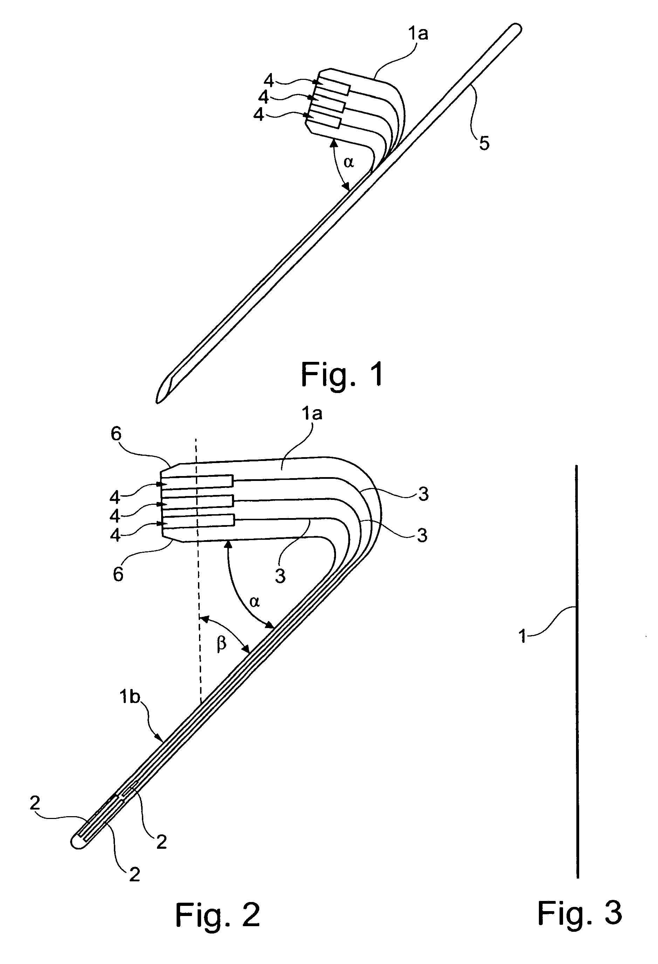

Der in den Figuren 1 bis 4 dargestellte Sensor für in-vivo Messungen hat ein flaches Substrat 1, das einen Sensorkopf 1a und einen von dem Sensorkopf 1a ausgehenden Sensorschaft 1 b bildet. Der Sensorschaft 1 b trägt Elektroden 2 für elektrochemische Messungen, die jeweils über Leiterbahnen 3 mit auf dem Sensorkopf 1a angeordneten Kontaktfeldern 4 verbunden sind.

Das Substrat 1 ist plan. Der Sensorschaft 1b und der Sensorkopf 1a haben deshalb eine coplanare Oberfläche, genauer gesagt zwei coplanare Oberflächen, nämlich die Oberseite und die Unterseite des Substrats 1, da das Substrat 1 aus einem Kunststoffblatt ausgeschnitten wurde. In einer Seitenansicht, also mit Blickrichtung senkrecht auf die Schmalseiten des Sensors, erscheint der Sensor deshalb als geradliniger Streifen, wie die in Figur 3 dargestellt ist.

Der Sensorkopf 1a steht seitlich von dem Sensorschaft 1b ab, so dass die Kontaktfelder 4 seitlich von dem Sensorschaft 1b angeordnet sind und die Leiterbahnen 3 auf dem Sensorkopf 1a nebeneinander quer zur Längsrichtung des Sensorschafts 1b verlaufen.

Zum Anschließen des Sensors an ein Messgerät kann der Sensorkopf 1a mit den Kontaktfeldern 4 in eine passende Buchse gesteckt werden. Vorteilhaft erfolgt die dabei erforderliche Steckbewegung wegen der Anordnung der Kontaktfelder 4 quer zur Längsrichtung des Sensorschafts 1 a. Eine solche Bewegung lässt sich für einen Benutzer leicht durchführen. Dabei lässt sich insbesondere die Gefahr vermeiden, dass der Sensor tiefer in den Körper hineingedrückt und dabei unnötig Schmerzen verursacht werden.

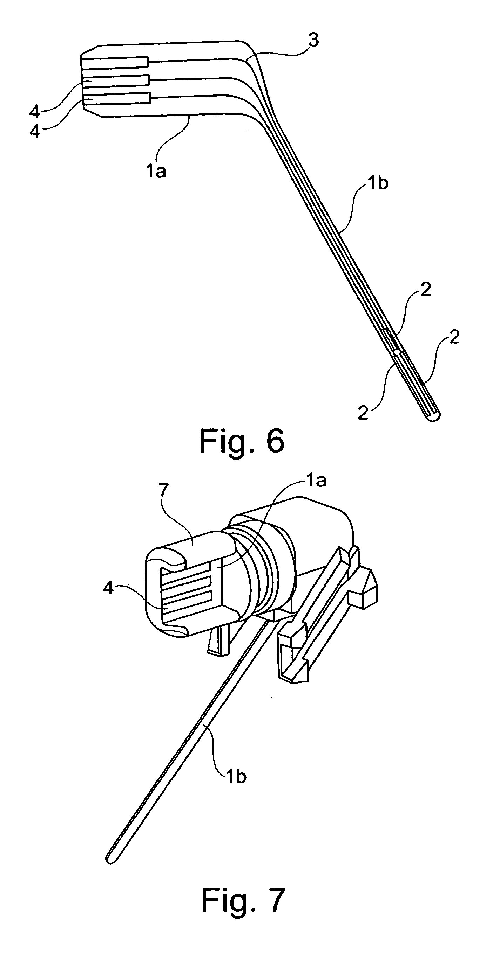

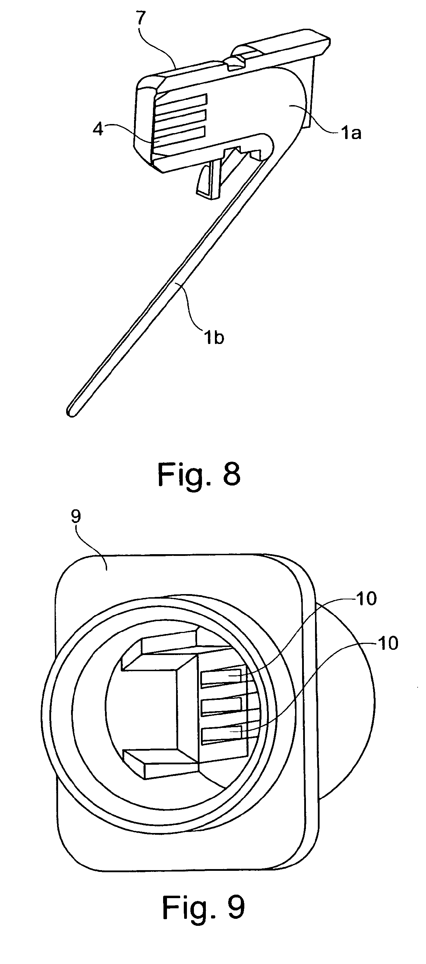

Bei einem dünnen und deshalb flexiblen Substrat kann das Aufstecken einer Buchse auf den Sensorkopf 1a manchen Benutzern Schwierigkeiten bereiten. Um dem abzuhelfen kann der Sensorkopf 1a vom Hersteller in einem Stützteil angeordnet werden, das zusammen mit dem Sensorkopf 1a einen elektrischen Steckverbinder ausbildet, dessen Verbindungsrichtung, also die Steckrichtung, in der ein dazu passender Steckverbinder zum Aufstecken bewegt werden muss, quer zur Längsrichtung des Sensorschaftes 1b verläuft. Ein Ausführungsbeispiel eines Sensors mit einem solchen Stützteil 7 ist in Figur 7 in einer Seitenansicht und in Figur 8 in einer Schnittansicht dargestellt. In Figur 9 ist zusätzlich ein Steckverbinder 9 dargestellt, der zu dem von dem Stützteil 7 und dem Sensorkopf 1a gebildeten Steckverbinder passt und zusammen mit dem Sensor ein System bildet. Der Steckverbinder 9 hat federnd bewegliche Linienkontakte 10, die sich in der Steckrichtung erstrecken und beim Schließen der Steckverbindung jeweils eines der Kontaktfelder 4 des Sensors kontaktieren. Der Sensorkopf 1a kann für sich alleine oder zusammen mit einem Stützteil 7 einen Steckverbinder bilden.

Ob mit oder ohne Stützteil kann der Sensorkopf 1a also durch eine Steckverbindung elektrisch an ein Messgerät angeschlossen werden. Die Kontaktfelder sind dabei derart auf dem Sensorkopf angeordnet, dass ein Steckverbinder zum Anschließen des Sensors an ein Messgerät nur durch eine quer zur Längsrichtung verlaufende Steckbewegung auf den Sensorschaft 1 b aufgesteckt werden kann.

Die durch die Anordnung der Kontaktfelder 4 auf dem Sensorkopf 1a vorgegebene Steckrichtung schließt mit der Längsrichtung des Sensorschafts 1b einen Winkel α ein. Die Kontaktfelder 4 sind nebeneinander in einer Reihe auf dem Steckteil 1a angeordnet. Die Steckrichtung ist quer zur Richtung der Reihe. Die Reihe der Kontaktfelder 4 schließt mit der Längsrichtung des streifenförmigen Sensorschafts 1b einen Winkel β ein, der in Figur 2 eingezeichnet ist.

Der Winkel α weicht bei dem dargestellten Ausführungsbeispiel um 20° bis 70°, insbesondere bei 30° bis 60°, von einem rechten Winkel ab, beträgt also im Falle eines spitzen Winkels 20° bis 70°, insbesondere 30° bis 60°, oder im Falle eines stumpfen Winkels 110° bis 160°, insbesondere 120° bis 150°. Bei dem dargestellten Ausführungsbeispiel ist der Winkel α ein spitzer Winkel. Der Sensorkopf 1a kann jedoch auch auf der gegenüberliegenden Seite von dem Sensorschaft 1 b abstehen, wie dies in Figur 6 dargestellt ist. In einem solchen Fall ist der Winkel α ein stumpfer Winkel. Der Winkel β weicht bei den dargestellten Ausführungsbeispielen ebenfalls um 20° bis 70°, beispielsweise 30° bis 60°, von einem rechten Winkel ab.

Der Sensorkopf 1a hat seitliche Einführschrägen 6, die einem Benutzer das Aufstecken eines nicht dargestellten Kupplungsteils oder bei der Herstellung das Aufstecken eines in den Figuren 7 und 8 dargestellten Stützteils erleichtern. Diese Einführschrägen 6 sind an gegenüberliegenden Seitenrändern des Sensorkopfes 1a angeordnet. Die Breite des Sensorkopfes 1a nimmt wegen der Einführschrägen 6 zu dem vorderen Ende, auf welches zum Anschließen des Sensors ein Steckverbinder aufgesteckt wird, hin ab.

Das Substrat 1 kann ähnlich wie eine Leiterplatte mit Metall beschichtet werden, um Leiterbahnen 3 und Kontaktfelder 4 auszubilden. Die Kontaktfelder 4 sind bevorzugt länglich ausgebildet und erstrecken sich quer zur Längsrichtung des Sensorschafts 1b, bei dem dargestellten Ausführungsbeispiel ist die Längsrichtung der Kontaktfelder deshalb zugleich auch die Steckrichtung. Vorteilhaft lässt sich so die Zuverlässigkeit eines über die Kontaktfelder 4 hergestellten elektrischen Kontakts erhöhen.

Bei dem dargestellten Ausführungsbeispiel sind eine Arbeitselektrode, eine Gegenelektrode und eine Referenzelektrode vorhanden, wobei eine Referenzelektrode nicht zwingend erforderlich ist. Diese Elektroden 2 sind bei dem dargestellten Ausführungsbeispiel auf derselben Substratseite angeordnet. Prinzipiell ist es jedoch auch möglich, eine oder mehrere Elektroden auf der gegenüberliegenden Substratseite, also auf dessen Rückseite, anzuordnen, beispielsweise um ein besonders kompaktes Design zu verwirklichen. Die Rückseite des Substrats 1 lässt sich aber beispielsweise auch dazu nutzen, dort weitere Elektroden anzuordnen. Davon abgesehen können aber auch auf einer Seite des Substrats 1 mehrere Arbeitselektroden angeordnet werden. Auf diese Weise kann ein Sensor geschaffen werden, der an unterschiedliche Konzentrationsbereiche angepasste Messsensitivitäten hat oder zur Messung unterschiedlicher Analyten verwendet werden kann.

Die Elektroden 2 sind in den Figuren schematisch rechteckig dargestellt, können aber jede beliebige Gestalt haben.

Das Substrat 1 kann kostengünstig aus Kunststoff hergestellt werden, beispielsweise aus einem Blatt ausgeschnitten wird. Das Substrat 1 ist deshalb plan und hat einen rechteckigen Querschnitt, wie dies in der Seitenansicht der Figur 3 zu sehen ist. Bei dem dargestellten Ausführungsbeispiel sind der Sensorkopf 1a und der Sensorschaft 1b gleich dick. Es ist aber auch möglich den Sensorkopf 1a und/oder einen an den Sensorkopf 1a anschließenden Teil des Sensorschafts 1b etwas dicker auszubilden als den die Elektroden 2 tragenden Teil des Sensorschafts. Auf diese Weise kann die Stabilität des Sensors erhöht werden, so dass sich eine Steckverbindung auch ohne ein den Sensorkopf 1a umgebendes Stützteil 7 leichter schließen lässt.

Der Sensorkopf 1a kann vorteilhaft länglich ausgebildet sein, um das Aufstecken eines Kupplungsteils zum Anschließen des Sensors an ein Mess- oder Auswertegerät oder eines Stützteils 7 zu erleichtern. Der Sensorkopf 1a schließt bei den gezeigten Ausführungsbeispielen mit dem geradlinig verlaufenden Sensorschaft 1b den Winkel α ein.

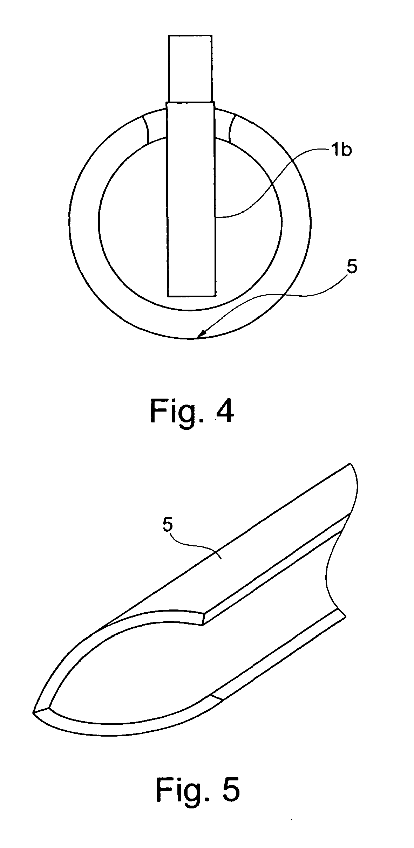

Zu dem Sensor gehört eine Kanüle 5, mit der das auf dem Sensorschaft 1 b angeordnete Elektrodensystem für transkutane Messungen in den Körper eines Patienten insertiert werden kann. Die Kanüle 5 hat einen in ihrer Längsrichtung verlaufenden Schlitz, in dem der Sensorschaft 1b hochkant angeordnet ist. Wie insbesondere Figur 4 zeigt, hat der Sensorschaft 1b bei dem dargestellten Ausführungsbeispiel eine Breite, die größer als der Innendurchmesser der Kanüle 5 ist, so dass der Sensorschaft 1b aus dem Schlitz herausragt. Es ist aber auch möglich, dass die Breite des Sensorschafts 1 b kleiner als der Kanülendurchmesser ist.

Da das Substrat 1 in dem Schlitz hochkant angeordnet ist, ist eine Schmalseite des Substrats 1 der dem Schlitz im inneren der Kanüle 5 gegenüberliegenden Innenfläche der Kanüle zugewandt. Die gegenüberliegende Schmalseite des Substrats 1, genauer gesagt des als Streifen ausgebildeten Sensorschafts 1b, ist von der Kanüle 5 angewandt.

Figur 5 zeigt das distale Ende der Kanüle 5. Wie darin dargestellt, hat die Kanüle 5 ein schräg angeschnittenes Ende, das in einer Spitze mündet. Der Schlitz ist dabei auf der Seite der Kanüle angeordnet, welche der die Spitze aufweisenden Seite gegen überliegt.

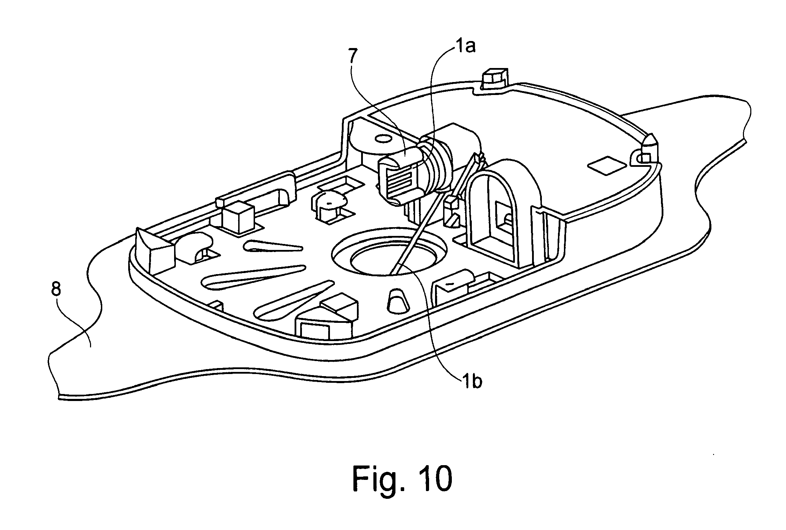

Der Sensor kann bei Gebrauch an einer Trägereinheit 8 befestigt sein, die auf den Körper eines Patienten aufgeklebt ist. Ein Beispiel einer solchen Trägereinheit 8 ist in Figur 10 dargestellt. Auf einer solchen Trägereinheit kann vorteilhaft auch eine Messeinheit befestigt werden, die den Sensor über eine Steckverbindung mit Strom versorgt.

### Bezugszahlen

| | |
|---|---|
| 1 | Substrat |
| 1a | Sensorkopf |
| 1b | Sensorschaft |
| 2 | Elektrode |
| 3 | Leiterbahn |
| 4 | Kontaktfeld |
| 5 | Kanüle |
| 6 | Einführschräge |
| 7 | Stützteil |
| 8 | Trägereinheit |
| 9 | Steckverbinder |
| 10 | Linienkontakte |

## Patentansprüche

1. Sensor für in-vivo Messungen, mit
einem Substrat (1), das plan ist und einen mehrere Elektroden (2) tragenden Sensorschaft (1b) sowie einen Sensorkopf (1a) bildet, der zum Anschließen des Sensors mit einem elektrischen Steckverbinder (9) kontaktiert wird und zur Kontaktgabe metallisierte Flächen als Kontaktfelder (4) trägt, die über Leiterbahnen (3) mit den Elektroden (2) verbunden sind und deren Anordnung eine Steckrichtung zum Aufstecken des Steckverbinders auf den Sensorkopf (1a) bestimmt,
der Sensorkopf (1a) vor Gebrauch des Sensors, wenn auf das flache Substrat (1) keine Biegekräfte wirken, seitlich von dem Sensorschaft (1b) absteht, so dass die Kontaktfelder (4) seitlich von dem Sensorschaft (1b) angeordnet sind,
wobei der Sensorkopf (1a) in einem Stützteil (7) angeordnet ist, das zusammen mit dem Sensorkopf (1a) einen elektrischen Steckverbinder (9) ausbildet,
**dadurch gekennzeichnet, dass**
die Steckrichtung des Steckverbinders (9) quer zur Längsrichtung des Sensorschafts (1b) verläuft.

2. Sensor nach Anspruch 1, **dadurch gekennzeichnet, dass** die Leiterbahnen (3) auf dem Sensorkopf (1a) nebeneinander quer zur Längsrichtung des Sensorschafts (1 b) verlaufen.

3. Sensor nach einem der vorstehenden Ansprüche, **dadurch gekennzeichnet, dass** der Sensorkopf (1a) Einführschrägen (6) aufweist.

4. Sensor nach einem der vorstehenden Ansprüche, **dadurch gekennzeichnet, dass** der Sensorkopf (1a) mit der Längsrichtung des Sensorschafts (1b) einen Winkel (α) einschließt, der um 20° bis 70°, bevorzugt um 30° bis 60°, von einem rechten Winkel abweicht.

5. Sensor nach einem der vorstehenden Ansprüche, **dadurch gekennzeichnet, dass** der Sensorkopf (1a) mit der Längsrichtung des Sensorschafts (1b) einen spitzen Winkel (α) einschließt

6. Sensor nach einem der vorstehenden Ansprüche, **dadurch gekennzeichnet, dass** die Kontaktfelder (4) länglich ausgebildet sind und sich quer zur Längsrichtung des Sensorschafts (1b) erstrecken.

7. Sensor nach einem der vorstehenden Ansprüche, **dadurch gekennzeichnet, dass** die Kontaktfelder (4) nebeneinander in einer Reihe angeordnet sind, die mit der Längsrichtung des Sensorschafts (1b) einen Winkel (β) einschließt, der um 20° bis 70°, bevorzugt um 30° bis 60°, von einem rechten Winkel abweicht.

8. Sensor nach einem der vorstehenden Ansprüche, **dadurch gekennzeichnet, dass** der Sensorschaft (1b) ein Streifen ist, dessen Breite größer als die Dicke des Substrats (1) ist.

9. Sensor nach einem der vorstehenden Ansprüche, **dadurch gekennzeichnet, dass** der Sensorkopf (1a) länglich ist und seine Längsrichtung quer zu dem Sensorschaft (1 b) orientiert ist.

10. Sensor nach einem der vorstehenden Ansprüche, **dadurch gekennzeichnet, dass** das Substrat (1) aus einem Kunststoffblatt ausgeschnitten ist.

11. Sensor nach einem der vorstehenden Ansprüche, **dadurch gekennzeichnet, dass** die Elektroden (2) auf einer Ober- und/oder einer Unterseite des Substrats (1) angeordnet sind.

12. Sensor nach einem der vorstehenden Ansprüche, **dadurch gekennzeichnet, dass** das Substrat in einer geschlitzten Kanüle steckt.

13. Sensor nach Anspruch 12, **dadurch gekennzeichnet, dass** die Kanüle (5) ein schräg angeschnittenes Ende aufweist, das in einer Spitze mündet, wobei der Schlitz auf der Seite der Kanüle (5) angeordnet ist, welche der die Spitze aufweisenden Seite gegen überliegt.

14. System mit einem Sensor nach einem der vorstehenden Ansprüche und einem Steckverbinder (9), der zum Anschließen des Sensors auf den Sensorkopf (1a) aufgesteckt wird und dabei eine elektrische Steckverbindung schließt, **dadurch gekennzeichnet, dass** der Steckverbinder (9) nur durch eine quer zur Längsrichtung des Sensorschafts (1b) verlaufende Steckbewegung auf den Sensorkopf (1 a) aufgesteckt werden kann.

## Claims

1. A sensor for in-vivo measurements having
a substrate (1) that is planar and forms a sensor shaft (1b) bearing multiple electrodes (2) as well as a sensor head (1a) that is to be connected to an electrical plug connector (9) in order to connect the sensor and bears metallized surfaces for contacting in the form of contact fields (4) that are connected by means of strip conductors (3) to the electrodes (2) and whose arrangement defines a plugging direction, in which the plug connector is to be plugged onto the sensor head (1a),
when there are no bending forces acting on the flat substrate (1) in an unused sensor the sensor head (1a) laterally protrudes from the sensor shaft (1b) such that the contact fields (4) are arranged aside the sensor shaft (1b),
wherein the sensor head (1a) is arranged in a support part (7), which, together with the sensor head (1a), forms an electrical plug connector (9),
**characterized in that**
the plugging direction of the plug connector (9) extends crosswise to the longitudinal direction of the sensor shaft (1b).

2. A sensor according to claim 1, **characterized in that** the strip conductors (3) run on the sensor head (1a) next to each other transverse to the longitudinal direction of the sensor shaft (1b).

3. A sensor according to any one of the preceding claims, **characterized in that** the sensor head (1a) comprises lead-in chamfers (6).

4. A sensor according to any one of the preceding claims, **characterized in that** the sensor head (1a) and the longitudinal direction of the sensor shaft (1b) form an angle (α) that deviates from a right angle by 20° to 70°, preferably by 30° to 60°.

5. A sensor according to any one of the preceding claims, **characterized in that** the sensor head (1a) and the longitudinal direction of the sensor shaft (1b) form an acute angle (α).

6. A sensor according to any one of the preceding claims, **characterized in that** the contact fields (4) are elongate in shape and run transverse to the longitudinal direction of the sensor shaft (1b).

7. A sensor according to any one of the preceding claims, **characterized in that** the contact fields (4) are arranged next to each other in a row, whereby the row and the longitudinal direction of the sensor shaft (1b) form an angle (ß) that deviates from a right angle by 20° to 70°, preferably by 30° to 60°.

8. A sensor according to any one of the preceding claims, **characterized in that** the sensor shaft (1b) is a strip whose width is larger than the thickness of the substrate (1).

9. A sensor according to any one of the preceding claims, **characterized in that** the sensor head (1a) is elongate in shape and has its longitudinal direction oriented crosswise to the sensor shaft (1b).

10. A sensor according to any one of the preceding claims, **characterized in that** the substrate (1) is cut from a sheet of plastic.

11. A sensor according to any one of the preceding claims, **characterized in that** the electrodes (2) are arranged on a top side and/or a bottom side of the substrate (1).

12. A sensor according to any one of the preceding claims, **characterized in that** the substrate is situated in a cannula having a slit.

13. A sensor according to claim 12, **characterized in that** the cannula (5) has a slanted end that ends in a tip, whereby the slit is arranged on the cannula's side that is opposite from the side comprising the tip.

14. System with a sensor according to any one of the preceding claims and a plug connector (9) that is to be plugged onto the sensor head (1a) in order to connect the sensor thereby closing an electrical plug connection, **characterized in that** the plug connector (9) can be plugged onto the sensor head (1a) only by means of a plugging motion that proceeds crosswise to the longitudinal direction of the sensor shaft (1b).

## Revendications

1. Capteur pour mesures in vivo, comprenant
un substrat (1) qui est plan et forme une tige de capteur (1b) portant plusieurs électrodes (2) ainsi qu'une tête de capteur (1a), qui est mise en contact avec un connecteur à fiches électrique (9) pour le raccordement du capteur et qui porte des surfaces métallisées en tant que champs de contact (4) pour l'établissement du contact, lesquelles surfaces sont reliées aux électrodes (2) par le biais de pistes conductrices (3) et dont l'agencement définit une direction d'enfichage pour la fixation du connecteur à fiches sur la tête de capteur (1 a),
la tête de capteur (1a) avant l'utilisation du capteur, lorsqu'aucune force de flexion ne s'exerce sur le substrat plat (1), s'écarte latéralement de la tige de capteur (1b), de sorte que les champs de contact (4) sont disposés latéralement par rapport à la tige de capteur (1 b),
la tête de capteur (1a) étant disposée dans une pièce de support (7), qui forme conjointement avec la tête de capteur (1a) un connecteur à fiches électrique (9), **caractérisé en ce que**
la direction d'enfichage du connecteur à fiches (9) est transversale à la direction longitudinale de la tige de capteur (1b).

2. Capteur selon la revendication 1, **caractérisé en ce que** les pistes conductrices (3) sont côte à côte sur la tête de capteur (1 a) transversalement à la direction longitudinale de la tige de capteur (1 b).

3. Capteur selon l'une des revendications précédentes, **caractérisé en ce que** la tête de capteur (1 a) présente des biseaux d'insertion (6).

4. Capteur selon l'une des revendications précédentes, **caractérisé en ce que** la tête de capteur (1a) forme avec la direction longitudinale de la tige de capteur (1b) un angle (α) qui s'écarte de 20° à 70°, de préférence de 30° à 60°, d'un angle droit.

5. Capteur selon l'une des revendications précédentes, **caractérisé en ce que** la tête de capteur (1a) forme un angle (α) aigu avec la direction longitudinale de la tige de capteur (1 b).

6. Capteur selon l'une des revendications précédentes, **caractérisé en ce que** les champs de contact (4) sont configurés de manière allongée et s'étendent transversalement à la direction longitudinale de la tige de capteur (1 b).

7. Capteur selon l'une des revendications précédentes, **caractérisé en ce que** les champs de contact (4) sont disposés côte à côte en une ligne qui forme avec la direction longitudinale de la tige de capteur (1b) un angle (β) qui s'écarte de 20° à 70°, de préférence de 30° à 60°, d'un angle droit.

8. Capteur selon l'une des revendications précédentes, **caractérisé en ce que** la tige de capteur (1 b) est une bande dont la largeur est supérieure à l'épaisseur du substrat (1).

9. Capteur selon l'une des revendications précédentes, **caractérisé en ce que** la tige de capteur (1 b) est allongée et sa direction longitudinale est orientée transversalement à la tige de capteur (1b).

10. Capteur selon l'une des revendications précédentes, **caractérisé en ce que** le substrat (1) est découpé dans une feuille de matière plastique.

11. Capteur selon l'une des revendications précédentes, **caractérisé en ce que** les électrodes (2) sont disposées sur le dessus et/ou le dessous du substrat (1).

12. Capteur selon l'une des revendications précédentes, **caractérisé en ce que** le substrat s'enfiche dans une canule à fente.

13. Capteur selon la revendication 12, **caractérisé en ce que** la canule (5) présente une extrémité découpée en biseau, qui aboutit à un embout pointu, la fente étant disposée sur le côté de la canule (5) qui se trouve en face du côté présentant l'embout pointu.

14. Système comprenant un capteur selon l'une des revendications précédentes et un connecteur à fiches (9), qui est fixé sur la tête de capteur (1a) pour le raccordement du capteur et ainsi ferme une connexion à fiches électrique, **caractérisé en ce que** le connecteur à fiches (9) ne peut être fixé sur la tête de capteur (1a) que par un mouvement d'enfichage transversal à la direction longitudinale de la tige de capteur (1 b).
